# DEMANDE DE BREVET EUROPEEN

(11) **EP 4 782 037 A1**
(43) Date de publication de la demande: **29.07.2026**
(21) Numéro de dépôt: 25223799.5
(22) Date de dépôt: 16.12.2025
(51) Int. Cl.: A61M 16/00, A61M 16/20

(54) **APPAREIL DE THÉRAPIE RESPIRATOIRE DÉLIVRANT UNE ASSISTANCE RESPIRATOIRE INTERMITTENTE PAR PRESSION POSITIVE**

(30) Priorité: 23.01.2025 FR 2500690
(71) Demandeur: EOVE, 64000 Pau (FR)
(72) Inventeur: DUCEUX, Stephane, 64000 Pau (FR); GALBRUN, Marie-Amedee, 64000 Pau (FR); JOURDAIN, Cedric, 64000 Pau (FR); LIU, Tinging, 64000 Pau (FR)
(74) Mandataire: Air Liquide

(57) **Abrégé**

L'invention concerne un appareil de thérapie respiratoire délivrant une assistance respiratoire intermittente par pression positive (IPPB) à une personne, comprenant une turbine (2), un circuit de gaz (3) interne avec un capteur de débit (5) et un dispositif anti-retour (6), un circuit patient (10) externe comprenant une valve expiratoire (11), une ligne de pilotage pneumatique (8) de la valve expiratoire (11) venant se raccorder entre la turbine (2) et le capteur de débit (5), et coopérant avec la valve expiratoire (11) pour contrôler l'ouverture ou la fermeture de ladite valve expiratoire (11), un capteur de pression proximale (7), et des moyens de pilotage (4) raccordés au capteur de pression proximale (7) et à la turbine (2), pour commander la turbine (2) en fonction des mesures opérées par le capteur de pression proximale (7) et le capteur de débit (5).

## Description

L'invention concerne un appareil de thérapie respiratoire délivrant une assistance respiratoire intermittente par pression positive ou IPPB (pour *Intermittent Positive Pressure Breathing* en anglais), aussi appelé « appareil IPPB ».

D'une façon générale, fournir une thérapie respiratoire de type IPPB (appelée « thérapie IPPB » ci-après) à certaines personnes, aussi appelés « patients », souffrant de problèmes ou pathologies respiratoires permet notamment d'améliorer :
- le drainage bronchique en délivrant un volume inspiratoire important permettant d'augmenter l'efficacité du débit expiratoire et de la toux ;
- la fonction respiratoire en allant au-delà de l'inspiration maximale du patient et en augmentant ainsi sa capacité vitale ; et
- le recrutement pulmonaire, en particulier des zones pulmonaires mal ou non ventilées.

Ainsi, EP4079356 enseigne un appareil d'assistance à la toux permettant d'opérer des insufflations de gaz aux patients souffrant de troubles respiratoires nécessitant une aide pour évacuer de leurs sécrétions pulmonaires et/ou pour améliorer leur élasticité pulmonaire et thoracique, que ces patients soient des patients adultes ou pédiatriques, lequel peut être utilisé facilement par une personne n'ayant pas ou peu de connaissances médicales, typiquement une auxiliaire de vie, une personne de l'entourage familial du patient, encore appelés « les aidants ».

D'une façon générale, la thérapie IPPB repose sur l'application d'un débit gazeux constant en inspiration jusqu'à l'atteinte d'une pression maximale, suivi d'une expiration avec ou sans pression expiratoire positive (PEP).

La synchronisation avec les phases respiratoires du patient se fait grâce à une détection de l'effort du patient, typiquement l'appel de gaz en début d'inspiration, ou par un mécanisme ou dispositif de déclenchement manuel de l'inspiration, par exemple via un appui sur un bouton ou une touche de commande.

Il existe plusieurs types de dispositifs ou d'appareils de thérapie respiratoire permettant de mettre en œuvre une thérapie IPPB mais ceux-ci présentent tous des inconvénients plus ou moins importants.

Ainsi, certains de ces dispositifs de thérapie IPPB comprennent un compresseur de gaz ou analogue couplé à une vanne de régulation du débit, ce qui les rend souvent bruyants et/ou limite leurs performances notamment du fait d'une chute de débit au-delà d'une pression donnée lors des phases inspiratoires du patient traité.

D'autres appareils existants mettent en œuvre une expiration passive des gaz par les patients, associée à une pression expiratoire positive (PEP), ce qui peut avoir comme inconvénient de limiter la profondeur de l'expiration du gaz par les patients.

D'autres encore combinent l'utilisation d'une ou plusieurs vannes proportionnelles pour piloter une valve d'expiration avec ou sans pression expiratoire positive (PEP), ce qui présente l'inconvénient d'une complexification de l'architecture globale de l'appareil avec une diminution de sa fiabilité, et une augmentation du coût de l'appareil.

Des appareils de thérapie respiratoire sont aussi décrits par DE102014001218, WO2015/110098, EP1502619 et US2013/047983. En particulier, enseigne un appareil de thérapie respiratoire comprenant une coque ou carcasse périphérique, une turbine motorisée alimentant un circuit de gaz interne comprenant un capteur de débit, un capteur de pression, un dispositif anti-retour et une valve expiratoire pilotée par une ligne de pilotage pneumatique, et des moyens de pilotage. Un circuit patient externe se raccorde fluidiquement au circuit de gaz interne pour convoyer le gaz jusqu'au patient.

Un problème est dès lors de proposer un appareil de thérapie respiratoire permettant de délivrer une assistance respiratoire intermittente par pression positive, c'est-à-dire une thérapie de type IPPB, ou « appareil IPPB », qui soit amélioré, notamment qui permette de palier tout ou partie des inconvénients susmentionnés, en particulier de délivrer une thérapie IPPB avec un confort accru pour le patient, notamment en termes de limitation du bruit généré, et/ou de meilleures performances en termes de précision de régulation du débit gazeux administré et ce, indépendamment du niveau de pression, et/ou préférentiellement une meilleure précision de détection de l'effort respiratoire du patient, donc de la synchronisation de la délivrance de gaz audit effort respiratoire du patient.

Une solution selon l'invention concerne un appareil (i.e. dispositif ou installation) de thérapie respiratoire, c'est-à-dire un « appareil IPPB », pour fournir une assistance respiratoire intermittente par pression positive (IPPB) à une personne, i.e. un patient, comprenant :
- une coque ou carcasse périphérique,
- une turbine motorisée pour fournir un gaz respiratoire sous pression,
- un circuit de gaz interne pour convoyer le gaz respiratoire délivré par la turbine,
- un capteur de débit agencé sur le circuit de gaz interne,
- un dispositif anti-retour agencé dans le circuit de gaz interne, en aval du capteur de débit.
- un circuit patient externe venant se raccorder fluidiquement au circuit de gaz interne pour convoyer le gaz respiratoire provenant du circuit de gaz interne,
- une valve expiratoire agencée sur le circuit patient externe,
- une ligne de pilotage pneumatique de la valve expiratoire venant se raccorder fluidiquement au circuit de gaz interne entre la turbine et le capteur de débit, et coopérant avec la valve expiratoire pour contrôler l'ouverture ou la fermeture de ladite valve expiratoire,
- un capteur de pression proximale agencé pour permettre une mesure de la pression au sein du circuit patient externe à proximité de la valve expiratoire,
- et des moyens de pilotage raccordés au capteur de pression proximale et à la turbine motorisée, et configurés pour commander la turbine en fonction d'au moins une mesure de pression opérée par le capteur de pression proximale et d'au moins une mesure de débit opérée par le capteur de débit,
et dans lequel la turbine, les moyens de pilotage, le circuit interne, le capteur de débit et le dispositif anti-retour sont agencés dans la coque ou carcasse périphérique.

De plus, dans l'appareil de l'invention, les moyens de pilotage sont configurés pour commander un ralentissement ou un freinage de la turbine lorsque lesdits moyens de pilotage déterminent que la pression proximale mesurée au plus près du patient atteint une valeur de pression maximale (pré)réglée ou qu'un temps maximum d'inspiration (pré)fixé est atteint, ledit ralentissement ou un freinage de la turbine conduisant à (i.e. engendrant) une chute de pression dans le circuit de gaz interne en amont du clapet anti-retour et dans la ligne de pilotage pneumatique de la valve expiratoire, et la valve expiratoire est configurée pour s'ouvrir en réponse à ladite chute de pression.

Selon le mode de réalisation considéré, l'appareil de l'invention, c'est-à-dire « l'appareil IPPB », peut comprendre l'une ou plusieurs des caractéristiques suivantes :
- les moyens de pilotage sont raccordés au capteur de pression proximale, c'est-à-dire que le capteur de pression peut être, selon le mode de réalisation considéré, relié électriquement auxdits moyens de pilotage et/ou être (directement) intégrés auxdits moyens de pilotage, de manière à coopérer avec lesdits moyens de pilotage.
- une ligne de pilotage pneumatique est une liaison pneumatique, typiquement un tuyau flexible, de préférence en polymère.
- de préférence, le capteur de pression proximale est intégré aux moyens de pilotage, en particulier porté par une carte électronique faisant partie des moyens de pilotage.
- le capteur de pression proximale comprend une ligne de mesure de pression venant se raccorder au circuit patient en aval de la valve expiratoire.
- le capteur de pression proximale est configuré pour permettre une mesure de la pression au sein du circuit patient externe entre la valve expiratoire et une interface respiratoire assurant les échanges gazeux avec le patient, typiquement lors des phases inspiratoires, et/ou éventuellement lors des phases expiratoires dudit patient.
- le circuit de gaz interne comprend un passage, un conduit ou analogue servant à véhiculer du gaz.
- l'interface respiratoire est ou comprend un masque respiratoire, une sonde de trachéostomie ou un embout buccal.
- le circuit de gaz interne comprend une sortie de gaz.
- le circuit patient externe vient se raccorder fluidiquement à ladite sortie de gaz du circuit de gaz interne.
- le circuit patient externe comprend un tuyau flexible, typiquement en polymère.
- le circuit patient externe peut aussi comprendre ou mettre en oeuvre des éléments de connexion fluidique additionnels de type connecteurs, raccords ou analogues.
- le circuit patient externe comprend une branche unique ou simple branche. Selon un autre mode de réalisation, il peut être à double branche.
- les moyens de pilotage comprennent au moins un microprocesseur,
- ledit au moins un microprocesseur est agencé sur au moins une carte électronique.
- les moyens de pilotage comprennent au moins une carte électronique.
- les moyens de pilotage comprennent un (micro)contrôleur.
- la turbine motorisée comprend un moteur électrique.
- le moteur électrique est alimenté électriquement (i.e. 110/220V).
- les moyens de pilotage sont configurés pour commander les accélérations du moteur électrique de la turbine.
- les moyens de pilotage sont configurés pour commander les décélérations dudit moteur. Par décélération, on entend un freinage du moteur, par exemple opéré par inversion des phases du moteur.
- le dispositif anti-retour comprend un clapet anti-retour, une valve anti-retour ou analogue.
- le dispositif anti-retour est configuré pour s'opposer à tout flux négatif, c'est-à-dire remontant en direction de la turbine, i.e. s'écoulant dans le sens opposé au sens normal de circulation du gaz de la turbine vers le patient.
- la valve expiratoire est préférentiellement une valve à 3 voies.
- selon un autre mode de réalisation, la valve expiratoire peut être une valve de PEP.
- le moteur électrique de la turbine est piloté, i.e. commandé ou contrôlé, par les moyens de pilotage.
- la ligne de pilotage pneumatique comprend un tuyau flexible en polymère de petit diamètre, typiquement d'environ 2 à 10 mm de diamètre.
- il comprend une coque ou carcasse périphérique formée d'une ou plusieurs sous-parties fixées entre elles, par exemple par vissage.
- la coque ou carcasse périphérique peut être en polymère rigide.
- il comprend une interface graphique utilisateur ou IGU.
- l'IGU peut comprend un écran d'affichage, de préférence de type tactile, et/ou des touches de sélection ou de validation, notamment des touches tactiles affichées sur l'écran.
- il comprend des moyens de mémorisation, telle une mémoire RAM ou EEPROM, ou une mémoire flash, ou autre.
- les moyens de pilotage sont configurés pour comparer la valeur de pression proximale mesurée par le capteur de pression proximale à la valeur de pression maximale (pré)réglée et en déduire une fin de phase inspiratoire ou un début de phase expiratoire du patient.
- les moyens de pilotage comprennent un compteur temporel permettant de déterminer lorsque le temps maximum d'inspiration (pré)fixé est atteint.
- le temps maximum d'inspiration (pré)fixé est mémorisé par les moyens de mémorisation.
- le temps maximum d'inspiration est (pré)fixé, i.e. réglé par l'utilisateur (i.e. personnel médical), via l'IGU.
- la valeur de pression maximale (pré)réglée est mémorisé par les moyens de mémorisation.
- la valeur de pression maximale est (pré)réglée via l'IGU, i.e. réglée par l'utilisateur (i.e. personnel médical).
- la valve d'expiration, i.e. valve expiratoire, est configurée pour s'ouvrir automatiquement dès lors qu'une dépression se crée dans le circuit patient du fait d'un freinage de la turbine, i.e. c'est-à-dire lorsque (le moteur de) la turbine ralentit. Ceci est possible grâce au raccordement fluidique de la ligne de pilotage de la valve d'expiration en aval de la turbine et en amont du dispositif anti-retour, c'est-à-dire entre turbine et dispositif anti-retour de gaz.
- la valve d'expiration comprend un corps de valve comprenant des moyens d'étanchéité flexibles.
- les moyens d'étanchéité flexibles comprennent une membrane, un ballonnet ou analogue, de préférence flexible et/ou déformable, typiquement une membrane.
- la ligne de pilotage pneumatique contrôle l'ouverture et/ou la fermeture de la valve d'expiration en agissant pneumatiquement, c'est-à-dire via une fourniture de gaz sous pression, sur les moyens d'étanchéité flexibles du corps de valve.
- le corps de valve de la valve d'expiration comprend au moins un orifice de mise à l'atmosphère.
- les moyens d'étanchéité flexibles sont configurés pour autoriser ou, à l'inverse, pour empêcher une circulation du flux gazeux au travers de l'orifice de mise à l'atmosphère, c'est-à-dire une décharge de gaz à l'atmosphère.
- les moyens d'étanchéité flexibles sont agencés sur un passage de flux gazeux traversant le corps de valve.
- les moyens d'étanchéité flexibles sont configurés pour coopérer avec au moins une partie de la paroi du corps de valve pour contrôler ou assurer l'étanchéité gazeuse, en particulier au moins une partie de la paroi du passage de flux gazeux agencé dans le corps de valve.

Dans le cadre de la présente invention:
- on appelle « capteur de pression », tout dispositif ou moyen permettant de déterminer une pression de gaz.
- on appelle « capteur de débit », tout dispositif ou moyen permettant de déterminer un débit de gaz, y compris indirectement via des mesures de pression.
- les termes « turbine », « compresseur », « soufflante » ou analogue sont considérés comme équivalents et substituables.
- les termes « valve » et « vanne » sont considérés comme équivalents et substituables.
- les termes « moyens » et « dispositif » sont considérés comme équivalents et substituables, par exemple les termes « moyens de pilotage » sont considérés comme équivalents et substituables par les termes « dispositif de pilotage ».
- les termes « piloté », « commandé » et « contrôlé » sont considérés comme équivalents et substituables.

L'invention va maintenant être mieux comprise grâce à la description détaillée suivante, faite à titre illustratif mais non limitatif, en référence aux figures annexées parmi lesquelles :
Fig. 1 est un schéma de principe d'un mode de réalisation d'un appareil de thérapie respiratoire selon l'invention.
Fig. 2 schématise (vue extérieure partielle) un mode de réalisation d'un appareil selon l'invention, tel celui de Fig. 1, avec son raccordement au secteur.
Fig. 3 schématise des courbes de débit (Q) et pression (P) obtenues avec un appareil IPPB selon l'art antérieur.
Fig. 4 schématise des courbes de débit (Q) et pression (P) obtenues avec un appareil IPPB selon l'invention.

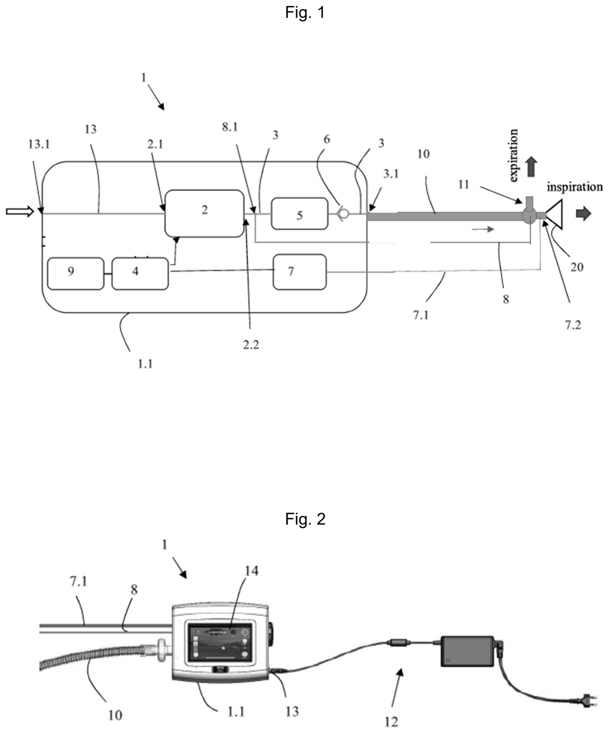

Fig. 1 et Fig. 2 schématisent un mode de réalisation d'un appareil ou dispositif 1 de thérapie respiratoire selon l'invention permettant de fournir une assistance respiratoire intermittente par pression positive, appelée « thérapie IPPB » ci-après, à une personne en ayant besoin, à savoir typiquement un patient à traiter ou analogue.

Comme illustré sur le schéma de principe de Fig. 1, l'appareil de thérapie respiratoire 1 ou « appareil IPPB » comprend une turbine 2 motorisée (aussi appelée (micro-)soufflante, pompe, compresseur ou analogue) combinée à un système de commande passif comprenant d'une valve d'expiration 11, aussi appelée valve expiratoire, et à des moyens de mesure de pression proximale, typiquement un capteur de pression proximale 7, comme décrit ci-après, dans le but de délivrer la thérapie IPPB au patient de manière efficace et avec un confort accru pour le patient, notamment en termes de limitation ou de réduction des nuisances sonores, c'est-à-dire du bruit généré lors du fonctionnement de l'appareil 1.

Plus précisément, la turbine 2 motorisée comprend un moteur électrique qui est piloté, i.e. contrôlé, par des moyens de pilotage 4, typiquement par un (des) microprocesseur agencé sur une (des) carte électronique. Elle est alimentée en air ambiant par une ligne d'amenée d'air 13, tel un conduit, un passage ou analogue, qui relie une entrée d'air 13.1 à l'entrée 2.1 de la turbine 2.

La turbine 2 motorisée a une architecture classique, à savoir qu'elle comprend schématiquement un moteur électrique agencé dans un carter de protection, lequel est surmonté d'une volute comprenant un compartiment interne dans lequel est agencée une roue à ailettes. La roue à ailettes est portée par l'axe du moteur. Elle est entrainée en rotation par l'axe du moteur, lors de son fonctionnement, de manière à d'aspirer le gaz respiratoire, tel de l'air, qui pénètre alors dans le compartiment interne de la volute, puis en ressort avant d'être envoyé vers le patient. Un exemple de turbine ou micro-soufflante pour appareil médical de ventilation est donné par EP2947328 mais, bien entendu, d'autres types de turbines peuvent convenir.

Utiliser une turbine motorisée 2 pour délivrer le gaz permet d'obtenir une limitation du bruit généré pendant le fonctionnement de l'appareil 1.

La turbine 2 fournit ensuite le gaz respiratoire sous pression, tel de l'air, à un circuit de gaz 3 interne servant à acheminer, i.e. convoyer, le gaz respiratoire délivré en sortie 2.2 de turbine 2 jusqu'à une interface respiratoire 20, tel un masque respiratoire, un embout buccal ou une sonde de trachéostomie, fournissant le gaz respiratoire aux voies aériennes du patient à traiter.

Afin de convoyer le gaz respiratoire provenant du circuit de gaz 3 interne, c'est-à-dire agencé dans la coque 1.1 de l'appareil 1, jusqu'à l'interface respiratoire 20, on prévoit un circuit patient 10 externe, typiquement un (des) conduit ou tuyau flexible, qui vient se raccorder fluidiquement à une sortie de gaz 3.1 du circuit de gaz 3 interne, typiquement un tuyau flexible en polymère. La sortie de gaz 3.1 peut être portée par un connecteur ou raccord de sortie agencé sur la carcasse 1.1 de l'appareil 1. Le circuit patient 10 externe vient se raccorder fluidiquement audit raccord de sortie, par exemple s'y emboiter, s'y viser, s'y fixer via un système à baïonnette ou tout autre système de raccordement adapté, de préférence le tuyau flexible comprend des moyens de connexion fluidique complémentaires du raccord de sortie.

Afin de mesurer le débit de gaz au sein de l'appareil 1, un capteur de débit 5 est agencé sur le circuit de gaz 3 interne, c'est-à-dire en aval de la sortie de la turbine 2 et en amont de la sortie de gaz 3.1 du circuit de gaz 3. Le capteur de débit 5 fournit les mesures de débit aux moyens de pilotage 4, où ils sont traités afin de réguler le fonctionnement de la turbine 2, typiquement les accélérations ou le freinage de son moteur électrique. Le capteur de débit 5 est raccordé électriquement aux moyens de pilotage 4, typiquement par des liaisons électriques, tels des câbles électriques ou analogues, afin de lui transmettre les mesures de débit réalisées.

On peut aussi utiliser ces mesures de débit pour vérifier le bon fonctionnement du dispositif anti-retour 6, e.g. clapet anti-retour, en s'assurant qu'il n'existe aucun flux négatif remontant vers la turbine 2, lors des phases expiratoires du patient, c'est-à-dire de vérifier que le dispositif anti-retour 6 garantit une étanchéité fluidique dans le sens négatif.

Le circuit patient 10 externe comprend, quant à lui, la valve expiratoire 11 servant à contrôler les rejets de gaz expirés par le patient vers l'atmosphère ambiante étant donné que cette valve expiratoire 11 permet de mettre en relation la partie aval du circuit patient 10 avec l'extérieur, c'est-à-dire l'atmosphère ambiante, en particulier lors des phases expiratoires du patient.

Par ailleurs, afin d'empêcher les remontées de gaz/pression et permettre leur meilleure évacuation via la valve expiratoire 11, un dispositif anti-retour 6, c'est-à-dire un clapet anti-retour, une valve unidirectionnelle ou analogue, est agencé sur le circuit de gaz 3 interne, en aval du capteur de débit 5 et en amont de la sortie de gaz 3.1 du circuit de gaz 3. Un tel dispositif anti-retour 6 présente en outre l'avantage d'être simple à mettre en œuvre et de ne demander aucun pilotage particulier, donc d'être aussi de coût bas.

La présence de ce dispositif anti-retour 6, typiquement un clapet anti-retour, permet aussi d'améliorer le fonctionnement de l'appareil 1, typiquement l'ouverture de la valve expiratoire 11 lors des phases expiratoires, comme expliqué ci-après.

La valve expiratoire 11, en particulier son ouverture et/ou sa fermeture, est commandée ou contrôlée par une ligne de pilotage pneumatique 8, tel un conduit flexible, venant se raccorder fluidiquement 8.1 au circuit de gaz 3 interne entre la turbine 2 et le capteur de débit 5. La ligne de pilotage pneumatique 8 coopère avec la valve expiratoire 11 en lui fournissant une pression gazeuse servant à contrôler l'ouverture ou la fermeture de ladite valve expiratoire 11, c'est-à-dire son degré d'ouverture/fermeture.

Préférentiellement, l'ouverture et/ou la fermeture de ladite valve expiratoire 11 est/sont contrôlée(s) par des moyens d'étanchéité flexibles sur lesquels agit la pression gazeuse apportée par la ligne de pilotage pneumatique 8, comme expliqué ci-après.

Enfin, le circuit patient 10 externe comprend aussi un capteur de pression proximale 7 agencé de manière à permettre une mesure de la pression au sein du circuit patient 10 et à proximité 7.2 de la valve expiratoire 11, typiquement entre la valve expiratoire 11 et l'interface respiratoire 20.

Ce capteur de pression proximale 7 permet de détecter les appels de gaz du patient, c'est-à-dire ses inspirations.

Le capteur de pression proximale 7 est raccordé aux moyens de pilotage 4, c'est-à-dire qu'il coopère avec ceux-ci, de sorte que ces derniers puissent piloter la turbine 2 à partir de tout ou partie des mesures opérées par le capteur de pression proximale 7. De préférence, le capteur de pression proximale 7 est directement intégré aux moyens de pilotage 4, par exemple porté par une carte électronique desdits moyens de pilotage 4.

L'utilisation d'une turbine motorisée 2 pilotée assure un confort accru tant au niveau du plus faible bruit généré par l'appareil 1 lors d'un traitement IPPB, que de l'adaptabilité du profil de débit délivré au patient, par exemple un débit en « rectangle » ou autre.

Par ailleurs, la mesure de la pression proximale, via le capteur de pression proximale 7 apporte une performance supplémentaire au dispositif 1, grâce à la grande précision des mesures de pression opérées, et aussi un confort d'utilisation pour le patient, grâce à une meilleure détection de l'effort respiratoire du patient, qui permettent d'obtenir une synchronisation efficace, voire (quasi-)optimale, entre appel de gaz par le patient et fourniture de gaz par la turbine lors des phases inspiratoires, et à l'inverse, le rejet des gaz expirés par la valve expiratoire 11.

L'appareil 1 comprend aussi des moyens de réglage ou de sélection, telles des touches virtuelles affichées par l'afficheur, i.e. écran digital 14, d'une interface graphique utilisateur 9 ou IGU. L'écran d'affichage 14 est de préférence de type tactile et/ou à affichage en couleurs. Il comprend des touches de sélection ou de validation, notamment des touches tactiles affichées sur l'écran.

La turbine 2, les moyens de pilotage 4, le circuit interne 3, le capteur de débit 5 et le dispositif anti-retour 6 sont agencés dans la coque ou carcasse 1.1 périphérique de l'appareil 1, par exemple une carcasse en polymère rigide ou un autre matériau adapté.

D'une façon générale, sont prévus aussi des moyens d'alimentation électrique, telle une liaison électrique secteur (110/220V), permettent d'alimenter électriquement l'appareil 1, en particulier tous les composants nécessitant de l'énergie électrique pour fonctionner, tels les moyens de pilotage 4, l'IGU 9, l'écran 14, les capteurs 5, 7... De préférence, comme illustré en Fig. 2, les moyens d'alimentation électrique peuvent comprendre un ensemble 12 comprenant un câble électrique avec transformateur de courant et prise de liaison au secteur venant se raccorder à un connecteur électrique 13 de l'appareil 1.

En outre, l'appareil 1 peut comprendre aussi des moyens de mise en route et/ou d'arrêt, comme une touche d'allumage/d'extinction (i.e. touche « On/Off » en anglais) ou équivalent.

Durant le fonctionnement de l'appareil 1, lors des phases inspiratoires du patient, la turbine 2 est pilotée pour délivrer un débit variable ou constant de gaz, selon le (les) réglage(s) ou paramètre(s) de contrôle entré(s), fixé(s) ou sélectionné(s) par l'utilisateur via l'IGU 9, en particulier une (des) valeur de pression maximale et/ou un temps maximum d'inspiration, c'est-à-dire une durée pendant laquelle du gaz est délivré au patient. On peut aussi sélectionner, selon le cas, d'autres paramètres, tel que débit d'inspiration, pente inspiratoire et/ou expiratoire, sensibilité de déclenchement, temps de thérapie, etc...

Les paramètre(s) de contrôle peuvent être mémorisés au sein de moyens de mémorisation de l'appareil 1, telle une mémoire flash, RAM, EEPROM ou analogue.

Le débit gazeux provenant de la turbine 2, pendant son fonctionnement, est délivré au patient après avoir été convoyé par le circuit de gaz 3 interne, donc via le capteur de débit 5 et le dispositif anti-retour 6 (i.e. clapet anti-retour ou analogue).

La ligne de pilotage pneumatique 8, i.e. tuyau flexible ou analogue, servant au pilotage de la valve expiratoire 11 est connectée au circuit de gaz 3 interne, en aval (en 8.1) de la sortie de la turbine 2, ce qui permet une fermeture de la valve expiratoire 11, typiquement par action sur les moyens d'étanchéité flexibles de la valve expiratoire 11, pendant l'inspiration du patient, grâce à la pression gazeuse provenant de la turbine 2 qui se propage via la ligne de pilotage pneumatique 8 jusqu'à la valve expiratoire 11.

Préférentiellement, les moyens d'étanchéité flexibles de la valve expiratoire 11 comprennent un ballonnet ou une membrane flexible/déformable ou analogue, et un orifice de mise à l'atmosphère ou évent en liaison avec l'atmosphère, pour contrôler le flux gazeux, c'est-à-dire pour bloquer toute sortie de gaz via ledit orifice de mise à l'atmosphère en empêchant ainsi toute sortie de gaz pendant les phases inspiratoires, ou à l'inverse libérer plus ou moins cet orifice pour autoriser alors le passage de gaz vers l'atmosphère, donc sa sortie du circuit patient 10 externe. Une telle architecture est classique.

Autrement dit, les moyens d'étanchéité flexibles sont agencés dans le corps de valve et coopèrent avec une partie de la paroi dudit corps de valve à la manière d'un clapet et d'un siège de clapet pour contrôler les flux gazeux au travers du corps de valve et leur libération à l'atmosphère.

Dans un mode de réalisation, la valve expiratoire 11, i.e. valve d'expiration, peut comprendre en outre une zone étanche comprise entre la membrane et le corps supérieur de la vanne 11 qui permet le pilotage en fonction de la pression qui est envoyée dans la zone étanche par le biais du piquage de pilotage.

Par ailleurs, la (les) mesure de pression proximale opérée(s) par le capteur de pression proximale 7 reflète(nt) la pression s'exerçant dans le circuit patient 10 au plus près du patient (en 7.2), c'est-à-dire au voisinage immédiat de ses voies respiratoires.

Quand la pression proximale mesurée au plus près du patient atteint la valeur de pression maximale (pré)réglée par l'appareil 1 ou quand le temps maximum d'inspiration (pré)fixé ou réglé est atteint, les moyens de pilotage 4 contrôlent la turbine 2, plus précisément son moteur électrique, de sorte que celle-ci ralentisse et/ou freine, c'est-à-dire que les rotations du moteur 2 diminuent et/ou stoppent, conduisant à une chute de pression dans le circuit pneumatique interne 3 en amont du clapet anti-retour 6.

Les comparaisons entre les valeurs de pression proximale mesurée et de pression maximale préfixée sont opérées par les moyens de pilotage 4, typiquement par le microprocesseur.

Par ailleurs, les moyens de pilotage 4 peuvent en outre comprendre un compteur temporel ou analogue pour déterminer la ou les durées des périodes d'inspiration de manière à pouvoir déterminer si le temps maximum d'inspiration est écoulé ou non. Ce dernier correspond à la durée s'écoulant entre le début d'une délivrance de gaz en réponse à la détection d'un début de phase inspiratoire du patient et la fin de la délivrance de gaz en réponse à la détection d'un début de phase expiratoire du patient, c'est-à-dire un arrêt de phase inspiratoire

Du fait de la présence du dispositif anti-retour 6, la chute de pression n'est pas directement transmise au circuit patient 10 mais reste dans le circuit interne 3 et dans la ligne de pilotage 8 de la valve expiratoire 11, ce qui permet à la valve 11 de s'ouvrir puisque la pression s'exerçant dans le ballonnet ou sur la membrane de la valve 11 diminue, et dès lors de laisser le gaz s'échapper à l'atmosphère, donc le patient expirer au travers de ladite valve expiratoire 11 qui communique alors fluidiquement avec l'atmosphère, via son orifice de mise à l'atmosphère étant donné que le ballonnet ou la membrane n'assure plus l'étanchéité gazeuse.

L'expiration du patient se déroule alors de façon passive à travers la valve d'expiration 11 du circuit patient 10 et vers l'atmosphère, i.e. au travers du corps de valve et de l'orifice de mise à l'atmosphère.

Dans le cadre de l'invention, l'utilisation de la pression proximale pour la synchronisation des phases inspiratoires et expiratoires du patient permet une meilleure sélectivité du système qui n'est plus influencée par le débit venant de la turbine 2 et par les résistances pneumatiques du circuit patient 10.

La détection de l'effort respiratoire du patient par l'appareil 1 se fait de manière plus précise et plus sensible, ce qui permet d'améliorer le traitement IPPB, notamment du fait d'une meilleure synchronisation de la délivrance de gaz à l'effort respiratoire du patient.

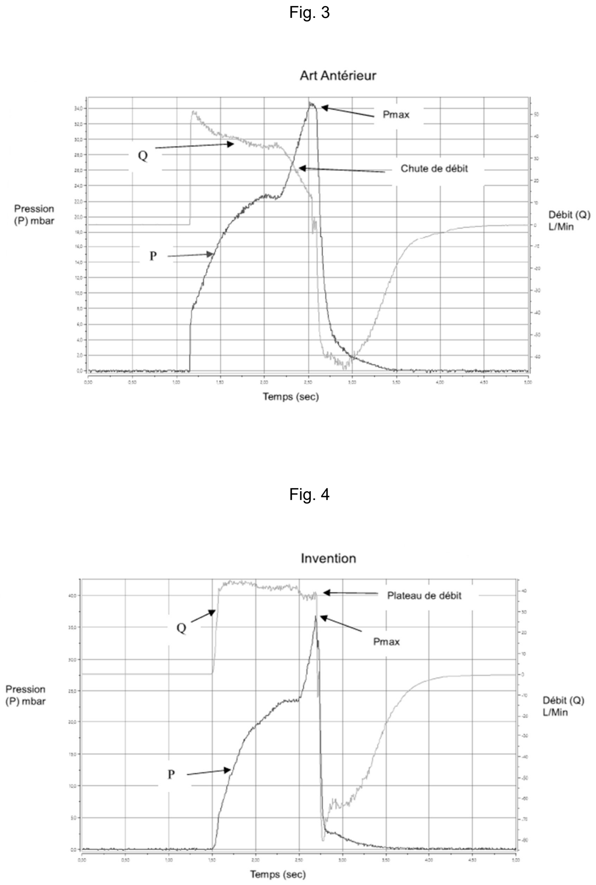

Par ailleurs, Fig. 3 et Fig. 4 schématisent des courbes de débit (Q) et pression (P) obtenues, au fil du temps (exprimé en secondes), avec, respectivement, un appareil IPPB selon l'art antérieur et, à titre comparatif, avec un appareil IPPB selon l'invention. La pression (P) est exprimée en mbar et le débit (Q) en L/min.

Comme on le voit sur Fig. 3, avec un appareil IPPB selon l'art antérieur, la chute de débit est progressive à partir du débit maximum (env. 50 L/Min ici) qui constitue un dépassement par rapport à la consigne de 40 l/min, et au fur et à mesure de l'augmentation de pression (P) qui augmente vers la pression maximale (Pmax) désirée, à savoir ici de l'ordre de 35 mbar. On voit même qu'on assiste à une chute brutale du débit (Q) dès lors que l'on se rapproche de la pression maximale (Pmax), c'est-à-dire même avant de l'avoir atteinte, et, lors du pic de pression (i.e. Pmax), le débit (Q) n'est plus que de 17 à 20 L/Min, soit une chute de 50% par rapport à la consigne de 40 l/min.

A l'inverse, comme illustré sur Fig. 4, avec un appareil IPPB conforme à la présente invention, tel celui décrit en Fig. 1 et Fig. 2, dans les mêmes conditions de fonctionnement, c'est-à-dire pour une pression maximale (Pmax) de l'ordre de 35 mbar, le débit ne chute pas à l'approche de la pression maximale (Pmax) mais est maintenu approximativement constant et à son maximum de l'ordre ici de 43 L/Min. Ceci se traduit par un « quasi-plateau » de débit jusqu'à atteindre la pression maximale Pmax.

Empêcher que le débit (Q) ne chute à l'approche de la pression maximale (Pmax) est un grand avantage car cela permet de délivrer une thérapie IPPB avec un confort accru pour le patient, notamment en termes de précision de régulation du débit gazeux administré et ce, indépendamment du niveau de pression.

D'une façon générale, l'appareil selon l'invention permet une régulation de débit plus précise et ce, indépendamment de la pression atteinte.

## Revendications

1. Appareil de thérapie respiratoire (1) pour fournir une assistance respiratoire intermittente par pression positive (IPPB) à une personne, comprenant :
- une coque ou carcasse périphérique (1.1),
- une turbine (2) motorisée pour fournir un gaz respiratoire sous pression,
- un circuit de gaz (3) interne pour convoyer le gaz respiratoire délivré par la turbine (2),
- un capteur de débit (5) agencé sur le circuit de gaz (3) interne,
- un dispositif anti-retour (6) agencé dans le circuit de gaz (3) interne, en aval du capteur de débit (5),
- un circuit patient (10) externe venant se raccorder fluidiquement au circuit de gaz (3) interne pour convoyer le gaz respiratoire provenant du circuit de gaz (3) interne,
- une valve expiratoire (11) agencée sur le circuit patient (10) externe,
- une ligne de pilotage pneumatique (8) de la valve expiratoire (11) venant se raccorder fluidiquement (8.1) au circuit de gaz (3) interne entre la turbine (2) et le capteur de débit (5), et coopérant avec la valve expiratoire (11) pour contrôler l'ouverture ou la fermeture de ladite valve expiratoire (11),
- un capteur de pression proximale (7) agencé pour permettre une mesure de la pression au sein du circuit patient (10) externe à proximité (7.2) de la valve expiratoire (11), et
- des moyens de pilotage (4) raccordés au capteur de pression proximale (7) et à la turbine (2) motorisée, et configurés pour commander les accélérations de la turbine (2) en fonction d'au moins une mesure de pression opérée par le capteur de pression proximale (7) et d'au moins une mesure de débit opérée par le capteur de débit (5),
et dans lequel la turbine (2), les moyens de pilotage (4), le circuit interne (3), le capteur de débit (5) et le dispositif anti-retour (6) sont agencés dans la coque ou carcasse périphérique (1.1),
**caractérisé en ce que** les moyens de pilotage (4) sont en outre configurés pour commander un ralentissement ou un freinage de la turbine (2) lorsque lesdits moyens de pilotage (4) déterminent que la pression proximale mesurée au plus près du patient atteint une valeur de pression maximale (pré)réglée ou qu'un temps maximum d'inspiration (pré)fixé est atteint, ledit ralentissement ou un freinage de la turbine (2) conduisant à une chute de pression dans le circuit de gaz (3) interne en amont du clapet anti-retour (6) et dans la ligne de pilotage pneumatique (8) de la valve expiratoire (11), et la valve expiratoire (11) est configurée pour s'ouvrir en réponse à ladite chute de pression.

2. Appareil selon la revendication 1, **caractérisé en ce que** le capteur de pression proximale (7) comprend une ligne de mesure de pression (7.1) venant se raccorder au circuit patient (10) en aval (7.2) de la valve expiratoire (11).

3. Appareil selon l'une des revendications 1 ou 2, **caractérisé en ce que** le capteur de pression proximale (7) est configuré pour permettre une mesure de la pression au sein du circuit patient (10) externe entre (7.2) la valve expiratoire (11) et une interface respiratoire (20) assurant les échanges gazeux avec le patient.

4. Appareil selon la revendication 1, **caractérisé en ce que** le circuit de gaz (3) interne comprend une sortie de gaz (3.1), le circuit patient (10) externe venant se raccorder fluidiquement à ladite sortie de gaz (3.1) du circuit de gaz (3) interne.

5. Appareil selon la revendication 1, **caractérisé en ce que** les moyens de pilotage (4) comprennent au moins un microprocesseur, de préférence agencé sur une ou plusieurs cartes électroniques.

6. Appareil selon la revendication 1, **caractérisé en ce que** le dispositif anti-retour (6) comprend un clapet anti-retour ou une valve unidirectionnelle.

7. Appareil selon la revendication 1, **caractérisé en ce que** les moyens de pilotage (4) sont configurés pour comparer la valeur de pression proximale mesurée par le capteur de pression proximale (7) à la valeur de pression maximale (pré)réglée et en déduire une fin de phase inspiratoire ou un début de phase expiratoire du patient.

8. Appareil selon la revendication 1, **caractérisé en ce que** les moyens de pilotage (4) comprennent un compteur temporel permettant de déterminer lorsque le temps maximum d'inspiration (pré)fixé est atteint.

9. Appareil selon la revendication 3, **caractérisé en ce que** l'interface respiratoire (20) comprend un masque respiratoire, une sonde de trachéostomie ou un embout buccal.

10. Appareil selon la revendication 1, **caractérisé en ce que** la turbine motorisée (2) comprend un moteur électrique piloté par les moyens de pilotage (4).

11. Appareil selon la revendication 1, **caractérisé en ce que** la valve expiratoire (11) est une valve à 3 voies ou une valve de PEP.

12. Appareil selon l'une des revendications 1 ou 11, **caractérisé en ce que** la valve expiratoire (11) comprend un corps de valve comprenant des moyens d'étanchéité flexibles.

13. Appareil selon la revendication 12, **caractérisé en ce que** les moyens d'étanchéité flexibles comprennent une membrane ou un ballonnet, de préférence flexible ou déformable.

14. Appareil selon les revendications 1 et 12 ou 13, **caractérisé en ce que** la ligne de pilotage pneumatique (8) contrôle l'ouverture ou la fermeture de la valve expiratoire (11) en agissant pneumatiquement, via une fourniture de gaz sous pression, sur les moyens d'étanchéité flexibles de la valve expiratoire (11).

15. Appareil selon la revendication 1, **caractérisé en ce que** le temps maximum d'inspiration ou la valeur de pression maximale est réglé par l'utilisateur via une interface graphique utilisateur (9) ou IGU de l'appareil (1).
